## Europäisches Patentamt

## European Patent Office

## Office européen des brevets

(19)

(11) Veröffentlichungsnummer: **0 139 844**
**B1**

(12) # EUROPÄISCHE PATENTSCHRIFT

(45) Veröffentlichungstag der Patentschrift:
23.09.87

(21) Anmeldenummer: **84107384.4**

(22) Anmeldetag: **27.06.84**

(51) Int. Cl.⁴: **A 23 J 3/00,** A 23 L 1/30,
A 23 K 1/04 //
A61K35/14, A61K7/00

(54) **Verfahren zur Verarbeitung von Tierblut und seinen Fraktionen.**

(30) Priorität: **28.10.83 PL 244395**

(43) Veröffentlichungstag der Anmeldung:
**08.05.85 Patentblatt 85/19**

(45) Bekanntmachung des Hinweises auf die Patenterteilung:
**23.09.87 Patentblatt 87/39**

(84) Benannte Vertragsstaaten:
**BE CH DE FR GB IT LI NL SE**

(56) Entgegenhaltungen:
**FR-A-2 334 306**

**CHEMICAL ABSTRACTS, Band 89, Nr. 15, Oktober 1978, Seite 462, Nr. 127880e, Columbus, Ohio, US; A.I. ZHARINOV et al.: "Use of precipitated blood plasma proteins in the production of canned meat paste products"**
**CHEMICAL ABSTRACTS, Band 99, Nr. 1, Juli 1983, Seite 413, Nr. 4397b, Columbus, Ohio, US**

**Die Akte enthält technische Angaben, die nach dem Eingang der Anmeldung eingereicht wurden und die nicht in dieser Patentschrift enthalten sind.**

(73) Patentinhaber: **Akademia Rolnicza, ul. Norwida 25, Wroclaw (PL)**

(72) Erfinder: **Zaleski, Stanislaw, ul. Canaletta 16/5, Wroclaw (PL)**
Erfinder: **Tereszkiewicz, Ryszard, ul. Wróblewskiego 25, Wroclaw (PL)**
Erfinder: **Kierzkowski, Marian, ul. Zachodnia 4/8, Wroclaw (PL)**
Erfinder: **Szubinska, Stanislawa, ul. Kazimierza Wielkiego 3/9, Lublin (PL)**
Erfinder: **Malicki, Adam, ul. Damrota 55/11, Wroclaw (PL)**
Erfinder: **Kumor, Leszek, ul. Wróblewskiego 25, Wroclaw (PL)**

(74) Vertreter: **Hansen, Bernd, Dr.rer.nat., Hoffmann, Eitle & Partner Patentanwälte Arabellastrasse 4, D-8000 München 81 (DE)**

## Beschreibung

Die Erfindung betrifft ein Verfahren zur Verarbeitung von Tierblut und seinen Fraktionen für Verbrauchs-, Futter-, pharmazeutische oder kosmetische Zwecke.

Die Blutstabilisierung als Reaktion auf den Erstarrungsprozess und die Erhaltung des Blutes im flüssigen Zustand wird unter anderem z. B. mit Hilfe von Natriumcitrat durchgeführt, das in 0,3 - 1,0 Massen-% zugegeben wird.

Eine solche Stabilisierung wird in den Betrieben durchgeführt, die entweder eine mechanisierte Blutsammlung durchführen oder als Vorprozess, wobei das Fraktionieren des Blutes der Schlachttiere zu Blutplasma und Satz von Blutkörperchen erfolgt. Da die Stabilisierung von Geflügelblut mit Natriumchlorid nicht wirksam ist, kann man mit der vorstehend beschriebenen Methode auch Geflügelblut stabilisieren.

Man verwendet gegenwärtig bei der Herstellung von Feinkornwürstchen die Fraktion von Schweine- und Rinderblut in Form von Blutplasma, wobei die Zugabe im flüssigen Zustand (üblich 5 - 20 Massen-%) je nach den gewünschten organoleptischen Eigenschaften der Würste erfolgt. Im Hinblick auf den mikrobiologischen Zustand muß die Verwendung von frischem Blutplasma zur Herstellung von Würsten binnen nur 2 Stunden nach der Gewinnung erfolgen.

Die Blutfraktion, die als Satz von Blutkörperchen anfällt und welche den Abfall nach der Plasmagewinnung bildet, wird im Prinzip nicht für Verbrauchs- und Futterzwecke verwendet, da der Zunahmekoeffizient negativ ist.

Das Geflügelblut wird in den Destruktoren (vorwiegend vom Hartmann-Typ) bei einer Temperatureinwirkung von ca. 403° K während ca. 30 Minuten behandelt, worauf eine Wasserabdampfung folgt. Man gewinnt ein Futtermehl mit einem niederen biologischen Wert, wodurch sich nur eine schwache Zuchtzunahme ergibt.

Aus PL-PS 122 519 ist ein Verfahren zur Härtung von Tierblut für Verbrauchs- und Futterzwecke bekannt, wobei 5 - 60 Massen-% Käsewasser oder Magermilch, 5 - 10 Massen-% Ferment von Gärungsmilchbakterien und 30 - 90 Massen-% Tierblut gemischt werden, und dieses Gemisch einer Temperatureinwirkung von 288 - 313° K bis zum Erhalt von mit Gel ausgefüllten Gerinnseln unterzogen wird. Ferner folgt ein Brühen im Wasser bei einer Temperatur von 343 - 383° K bis die ganze Masse eine schokoladen-braune Farbe annimmt.

In PL-PA 238 875 wird ein Verfahren zur Gewinnung eines Gelgerinnsels für Verbrauchs- und Futterzwecke unter Ausnutzung des stabilisierten Blutes, des Käsewassers, der Sauermilch oder deren Gemisch beschrieben, das in der Zugabe von Tiergalle in einer Menge von 0,1 - 5 Massen-% und von hefenabgeleiteten Substanzen in einer Menge von 1,0 - 15 Massen-% sowie Verbrauchszubereitungen zum Geschmack zu diesem Gemisch besteht: Das Gelgerinnsel kann in Formverpackungen hergestellt werden. Das auf diese Weise hergestellte Gelgerinnsel eignet sich im frischen Zustand zum direkten Verzehr nach Braten in einem Ölbad unter Zugabe von Geschmackszutaten. Es besitzt den Geschmack von Leber.

Die vorstehend beschriebenen Verfahren sind dadurch charakterisiert, daß in der Praxis die Herstellung des Produktes nur aus dem Gemischteil erfolgt, wodurch sich ökonomische Verluste und Nahrungsverluste ergeben, da ein Massenteil nicht verbunden war und eine entsprechende Blutmenge ausgeflossen ist. Dieser Effekt erinnert an den bekannten Effekt der Retraktion, der während des Erstarrens des soeben von den Gefäßen entfernten Blutes erfolgt und der darin besteht, daß es in dem dreidimensionalen Fibrinnetz, in dessen Maschen die flüssigen und geformten Blutkomponenten stecken, die das Gerinnsel bilden, nach einiger Zeit zu einem Schrumpfen von Fasern und zum Pressen von Heilserum kommt.

Die Erfindung betrifft ein Verfahren zur Verarbeitung von Tierblut und seinen Fraktionen, wobei das Blut thermisch behandelt wird und außerdem Blut, das mit Verbindungen, die die Calciumionen binden, stabilisiertn wird, wobei diese Ionen in natürlicher Weise im Blut vorkommen, insbesondere Natriumcitrat, oder eine Fraktion des so stabilisierten Blutes oder ein absichtlich auf diese Weise stabilisiertes Blut verwendet wird. Das Wesen der Erfindung besteht darin, daß die organischen oder anorganischen Calciumverbindungen in Mengen von 0,009 - 0,018 mol/dm$^3$ eingeführt werden, was 0,1 - 0,2 Massen-% Calciumchlorid entspricht, und man Natrium-, oder Kaliumsalze in Mengen von 0,085 - 0,43 mol/dm$^3$ im Verhältnis zur Blutmasse zugibt, was 0,5 - 2,5 Massen-% Natriumchlorid entspricht, worauf die Anordnung für die zum Produkterstarren erforderliche Zeit in dem ganzen Volumen belassen und dann das Produkt der thermischen Härtung unterzogen wird.

Es ist vorteilhaft, durch Zuführung von Eiweißkörper-Kohlenhydratsubstanzen mit neutraler oder saurer Reaktion die Nahrungswerte des Produkts zu bereichern und die Korrektur seines Geschmacks durchzuführen. Zu diesem Zweck werden auch Vitamine, Aminosäuren, Mikroelemente oder Geschmacks- und Geruchssubstanzen eingeführt. Da der häufigste Prozess der thermischen Härtung und der Verminderung von Bakterien die Pasteurisierung ist, ist es vorteilhaft, den pH-Wert auf maximal 6,5 einzustellen, um die Bildung toxischer Verbindungen aus dem Blut zu vermeiden. Für eine bessere und leitungsfähigere Produktdarstellung ist insbesondere ein pH-Wert bis 5,7 vorteilhaft.

Es wurde festgestellt, daß mit dem Verfahren gemäß der Erfindung das Geflügelblut in vorteilhafter Weise für Futterzwecke, als Satz von Blutkörperchen, für pharmazeutische oder kosmetische Zwecke genutzt werden kann. Darüber hinaus kann man das Vollblut und Plasma von Schlachttieren auf eine günstigere Weise als in den bis jetzt bekannten Verfahren verarbeiten. Die Einführung von stabilisierenden Verbindungen, deren Wirkung in der Blockierung der natürlich im Blut auftretenden Calciumkationen, i.e. Polyphosphate oder Natriumcitrat besteht, in das Blut bedingt das Funktionieren des Blutes der Schlachttiere und verhindert in diesem Zustand die Möglichkeit seiner Destabilisierung zwecks Erhaltung des Produkts von demselben Blut in erstarrter Form. Mit dem Verfahren gemäß der Erfindung bildet man die Bedingungen seiner Destabilisierung durch Zugabe von Calciumverbindungen, was die Wiederherstellung der Möglichkeit von Bluterstarren und die Darstellung des typischen Gerinnsels mit seinen Folgen, Retraktion und Teilausnutzung von Komponenten zur Darstellung des Produkts ermöglicht. Es wurde ganz unerwartet festgestellt, daß die durchgeführte Einführung von Calciumkationen im Verfahren gemäß der Erfindung in höheren Konzentrationen als notwendig für die Blutdestabilisierung oder eine Einführung eines Gemisches von Calciumkationen mit Natrium- oder Kaliumsalzkationen in der ausgewählten Konzentration den Prozess des typischen Erstarrens modifiziert, wodurch man die Folge gewinnt, die im voraus nicht vorzusehen war, die in der Darstellung des Netzes des aufgequollten Fibrins besteht, in welchem Bereich die Komponenten enthalten sind, die vom Ausgangsstoff abhängig sind. In diesem Netz sind im Falle von Verarbeitung des Tiervollblutes und Satz von Blutkörperchen die morphotischen Elemente enthalten, die der flüssige Blutteil umfaßt. Im Fall der Plasmaverarbeitung ist sein ganzer flüssiger Teil enthalten. Bei der Zugabe von Lösungen der bereichernden oder korrigierenden Verbindungen der Eigenschaften des Fertigprodukts kommt es zu dem analogen Effekt mit dem Unterschied, daß in dem Netz des aufgequollten Fibrins außerdem eine eingeführte Zugabe enthalten ist. Bei Anwendung von Vollblut hat das Rohprodukt eine lebendigrote Farbe, bei Anwendung von Plasma eine rosigweiße Farbe und bei Anwendung des Satzes von Blutkörperchen eine dunkelrote Farbe, wobei seine Konsistenz einer harten Gallerete ähnlich ist. Während der Blutverarbeitung mit dem Verfahren gemäß der Erfindung ist der Retraktionseffekt eliminiert, das Produkt entsteht aus dem vollen Volumen von Komponenten nicht nur aus Vollblut, sondern auch aus dem Satz von Blutkörperchen und aus Plasma sowie auch den Zugaben. Die Einwirkung der Wirkung der erhöhten Temperatur auf das Rohprodukt bewirkt eine Veränderung seiner Struktur durch Koagulation von Eiweißkörpern und Gewinnung einer Konsistenz, die der Konsistenz des Fertigeiweißes des Hühnereis ähnlich ist bei Verarbeitung von Vollblut und dem Satz von Blutkörperchen. Bei Plasmavorwärmung ist die entstandene Konsistenz minder brüchig und die Bindung ist stärker, was sich aus der wesentlich besseren Viskosität des Produkts ergibt. Trotzdem läßt es sich leicht zerkleinern sowie homogenisieren. Ganz unerwartet wurde festgestellt, daß der Prozess der thermischen Verarbeitung zum Eindicken von Eiweißkörpern des auf diese Weise dargestellten gehärteten Produkts aus Plasma führt, wodurch aus Rohplasma, das zirka 7 Massen-% Eiweiß in dem Fertigprodukt enthält, 11 - 12 Massen-% von Eiweiß gewonnen werden. Nach Behandlung des Rohprodukts durch thermische Prozesse verändert das Produkt aus Vollblut die Farbe zur braunen Farbe, aus Plasma zur weißen oder rosig-grauen Farbe und aus dem Satz von Blutkörperchen zur schwarzen Farbe. Bei Verarbeitung des Vollbluts von Schlachttieren nach dem Verfahren gemäß der Erfindung ist mit technologischer Leichtigkeit die Blutsammlung bei der gegenwärtigen Ausrüstung in den Fleischbetrieben zu realisieren. Falls es sich um Geflügelblut handelt, stellt man das fertige Futterprodukt dar, das als Nahrungswert Zuchteffekte, die um 50 % höher sind als das bis jetzt dargestellte Mehl, in den Destruktoren ergibt.

Die charakteristische Eigenschaft des Futterprodukts, das aus Schlachttierblut und aus Geflügel dargestellt wurde, ist die Gewinnung der besseren Zuchteffekte, wobei der Futterverbrauch minder sein soll. Die Verarbeitung von Plasma nach dem Verfahren gemäß der Erfindung und die Gewinnung des Produkts in erstarrter Form ermöglicht seinen Verbrauch zur Verarbeitung in vielfacher Weise. In der Fleisch-, Fisch- und Geflügelindustrie findet das Plasmaprodukt als Zugabe zu allen zerkleinerten Produkten Anwendung: Würste, Hamburger oder Pasteten. Bei Anwendung des Produkts zu diesen Fabrikaten wird das Wasser in der Menge, die der Wasseraufnahmefähigkeit des Muskelgewebes entspricht, ausgenutzt, wodurch die Produktleistung um die Masse des angewendeten Texturprodukts aus Plasma erhöht ist. Eine höhere Viskosität des Texturprodukts aus Plasma ermöglicht seine Anwendung in vielen Bereichen der Lebensmittelindustrie, z. B. Nudel- oder Kälteindustrie als Eierersatz. In der Form, die mit dem Verfahren nach der Erfindung gewonnen wurde, hat das Produkt aus Plasma eine erheblich verlängerte Beständigkeit; es eignet sich zur weiteren Verarbeitung noch nach zwei Wochen seit der Herstellungszeit, falls es unter Kältebedingungen aufbewahrt wird. Das Produkt aus Plasma kann auch gefroren sein und in diesem Zustand in die Kuterfleischmasse eingeführt sein, was das Eis ersetzt, das zur Abkühlung im Laufe der Zerkleinerung zugegeben wird. Der bis jetzt lästige Abfluß in Form von Satz von Blutkörperchen, der beim Fraktionieren von Vollblut entsteht, wird mit dem Verfahren gemäß der Erfindung zum wertvollen pharmazeutischen und kosmetischen Rohstoff verarbeitet, der neben den anderen Wertkomponenten 3,5 - 4 Eisenmassen-% enthält, das vor allem zweiwertig ist und vorzugsweise durch den Menschen- und Tierorganismus aufgenommen wird.

**Beispiel 1**

In das Gerät mit einem Volumen von 100 l werden 240 g Natriumcitrat eingeschüttet, worauf 60 l des soeben von den Gefässen entfernten Entenblutes eingegossen werden. Separat in 40 l Entenblut werden 800 g Natriumchlorid, 150 g Calciumchlorid (aufgelöst) und Methionin in einer Menge von 6 g und 350 ml 50 %ige Milchsäure zugegeben, dann in das erste Gerät gegossen und gemischt (30 - 60 Minuten bei einer Temperatur von 295° K). Nach dieser Zeit wird das Produkt aus dem vollen Volumen von Komponenten dargestellt, das die Form von harter Gallerte mit der roten Farbe besitzt. Das Rohprodukt wird in Stücke mit einer Dicke von ca. 40 mm geschnitten und im Wasser mit einer Temperatur von 353 - 363° K 30 - 40 Minuten pasteurisiert, wodurch die Farbe zur braunen Farbe an dem ganzen Durchschnitt von Stücken verändert wird.

Dieses Produkt ist für Futterzwecke für Pelztiere bestimmt.

**Beispiel 2**

In das Gerät mit einem Volumen von 100 l werden 80 l Rinderblut eingeführt, das durch Natriumcitrat in einer Menge von 0,5 Massen-% stabilisiert wird und mit Wasser auf ein Volumen von 100 l ergänzt wird, in welchem vorher 100 g Calciumchlorid, 1200 g Natriumchlorid und 2 g beliebige Vitamine, die in Wasser auflösbar sind, aufgelöst worden waren. Das Ganze wird 120 Minuten bei 295° K belassen. Das Rohprodukt wird geschnitten und pasteurisiert analog wie im Beispiel 1.

Das Produkt ist für Futterzwecke für Geflügel bestimmt.

**Beispiel 3**

In das Gerät mit einem Volumen von 100 l werden 50 l Schweineblut gesammelt, das mit Natriumcitrat in einer Menge von 0,5 Massen-% stabilisiert wird. In einem separaten Gerät werden in 50 l Wasser 100 g Calciumchlorid, 1300 g Natriumchlorid und 350 ml 50 %ige Milchsäure aufgelöst. Die auf diese Weise vorbereitete saure Lösung wird in das Gerät mit Blut eingegossen und das Ganze für 90 Minuten bei 295° K belassen. Das Rohprodukt wird in Stücke von ca. 100 g geschnitten und bei einer Temperaturvon 353 - 363° K pasteurisiert und für pharmazeutische oder Verbrauchszwecke bestimmt. Das Produkt kann im gefrorenen Zustand oder im Kühlhaus aufbewahrt werden.

**Beispiel 4**

In das Gerät mit einem Rauminhalt von 100 l wird das zentrifugierte Rinderplasma in einer Menge von 85 l eingegossen; dann wird es mit der wässrigen Lösung von 1200 g Natriumchlorid, Calciumchlorid und 5 g Zitronensaure zum Volumen von 100 l ergänzt. Das Ganze bleibt für 60 Minuten bei einer Temperatur von 295° K.

Nach dieser Zeit wird das Produkt von dem vollen Volumen von Komponenten dargestellt.

Das Rohprodukt wird in Stücke geschnitten und pasteurisiert wie im Beispiel 1. Nach Pasteurisierung verändern diese Stücke die Farbe von rosig-weiß zu weiß. Das Produkt ist für verbrauchszwecke bestimmt, z.B. zur Zugabe zu den zerkleinerten Verzehrprodukten.

**Beispiel 5**

In ein Gerät mit einem Rauminhalt von 100 l wird ein Satz von Schweineblutkörperchen in einer Menge von 85 l eingegossen. In einem separaten Gerät wird eine wässrige Lösung aus 120 g Calciumchlorid, 300 g Calciumkarbonat, 100 g Natriumchlorid und 5 g Zitronensäure in 15 l Wasser hergestellt. Diese Lösung wird in das Gerät mit dem Satz von Blutkörperchen eingegossen und 60 Minuten bei einer Temperatur von 292° K belassen. Nach dieser Zeit wird das Produkt von dem vollen Volumen von Komponenten dargestellt. Das Produkt wird in Stücke von einer Dicke von 40 mm geschnitten und pasteurisiert. Es ist für pharmazeutische und kosmetische Zwecke bestimmt.

**Patentansprüche**

1. Verfahren zur Verarbeitung von Tierblut und seinen Fraktionen durch thermische Behandlung, wobei man Blut mit in demselben natürlich vorkommenden Verbindungen, die die Calciumionen binden, insbesondere mit Natriumcitrat oder der Fraktion des auf diese Weise stabilisierten Blutes, oder Blut, das absichtlich auf diese Weise stabilisiert wurde, verwendet, dadurch gekennzeichnet, daß organische oder anorganische Calciumverbindungen in einer Menge von 0,009 - 0,018 mol/dm$^3$ eingeführt werden, was 0,1 - 0,2 Massen-% Calciumchlorid entspricht, und Natriumsalz oder Kaliumsalz in einer Menge von 0,085 - 0,43 mol/dm³ im Verhältnis zur Blutmasse zugegeben wird, was 0,5 - 2,5 Massen-% Natriumchlorid entspricht; dann wird das Gemisch für die erforderliche Zeit zum Erstarren des Produktes in dem vollen Volumen belassen, worauf das Produkt der thermischen Härtung unterzogen wird.
Substanzen eingeführt werden.

3. Verfahren nach Anspruch 1, dadurch gekennzeichnet, daß die Zugaben mit der saurem Reaktion in Form von Eiweißkörper-Kohlenhydrat-Substanzen eingeführt werden, wodurch die Endreaktion auf einen pH-Wert größer als 5, 7 beschränkt wird.

4. Verfahren nach Anspruch 1, dadurch gekennzeichnet, daß Vitamine, Aminosäuren und Mikroelemente zugegeben werden.

5. Verfahren nach Anspruch 1, dadurch gekennzeichnet, daß Geschmacks- und Geruchssubstanzen zugegeben werden.

6. Verfahren nach Anspruch 2, dadurch gekennzeichnet, daß organische oder anorganische Säure zur Einstellung eines pH-Wertes im Bereich von 5,7 - 6,5 zugegeben wird.

7. Verfahrem nach Anspruch 3, dadurch gekennzeichnet, daß sich der pH-Wert auf 6,5 beschränkt.

## Claims

1. Process for working up animal blood and its fractions by thermal treatment wherein blood containing compounds naturally occurring therein that bind the calcium ions, particularly with sodium citrate or with the fraction of blood stabilized in this way, or blood that was intentionally stabilized in this way is used, characterized in that organic or inorganic calcium compounds are introduced in an amount of 0.009 - 0.018 mol/dm³, which corresponds to 0.1 - 0.2 wt.% calcium chloride, and sodium salt or potassium salt are added in an amount of 0.085 - 0.43 mol/dm³ with respect to the blood mass, which corresponds to 0.5 - 2.5 wt.% sodium chloride; and then the mixture is left for the time required for the product to congeal in the full volume, whereupon the product was subjected to thermal hardening.

2. Process according to claim 1 characterized in that the added materials in the neutral reaction are introduced in the form of protein-carbohydrate substances.

3. Process according to claim 1 characterized in that the added materials in the acid reaction are introduced in the form of protein-carbohydrate substances whereby the end reaction is restricted to a pH value greater than 5.7.

4. Process according to claim 1 characterized in that vitamins, amino acids and microelements are added.

5. Process according to claim 1 characterized in that flavouring and aroma substances are added.

6. Process according to claim 2 characterized in that organic or inorganic acids are added for adjusting the pH value in the range of 5.7 - 6.5.

7. Process according to claim 3 characterized in that the pH value is restricted to 6.5.

## Revendications

1. Procédé pour le traitement de sang d'animaux et de ses fractions par traitement thermique, selon lequel on utilise du sang avec des composés qu'il contient naturellement, qui fixent les ions calcium, en particulier avec du citrate de sodium, ou la fraction du sang stabilisé de cette manière, ou du sang qui a été stabilisé intentionnellement de cette manière, caractérisé en ce que l'on introduit des composés organiques ou inorganiques de calcium en quantité de 0,009-0,018 mol/dm³, ce qui correspond à 0,1-0,2 % en poids de chlorure de calcium, et un sel de sodium ou un sel de potassium en quantite de 0,085-0,43 mol/dm³ par rapport à la masse de sang, ce qui correspond à 0,5 à 2,5 % en poids de chlorure de sodium; on abandonne ensuite le mélange pendant la durée nécessaire pour la coagulation du produit dans tout le volume, après quoi on soumet le produit au durcissement thermique.

2. Procédé selon la revendication 7, caractérisé en ce que les additifs à réaction neutre sont introduits sous forme de substances protéiniques-hydrate de carbone.

3. Procédé selon la revendication 7, caractérisé en ce que les additifs à réaction acide sont introduits sous forme de substances protéiniques-hydrates de carbone, ce qui limite la réaction finale à un pH supérieur à 5,7.

4. Procédé selon la revendication 1, caractérisé en ce que l'on ajoute des vitamines, des aminoacides et des oligo-éléments.

5. Procédé selon la revendication 1, caractérisé en ce que l'on ajoute des substances aromatiques et odorantes.

6. Procédé selon la revendication 2, caractérisé en ce que l'on ajoute un acide organique ou inorganique pour le réglage d'un pH dans l'intervalle de 5,7 - 6,5.

7. Procédé selon la revendication 3, caractérisé en ce que le pH est limité à 6,5.